# EUROPEAN PATENT APPLICATION

(11) **EP 4 424 342 A1**
(43) Date of publication of application: **04.09.2024**
(21) Application number: 22887217.2
(22) Date of filing: 31.10.2022
(51) Int. Cl.: A61M 5/158, A61M 1/36, B21D 22/26, B21G 1/08

(54) **HOLLOW MEDICAL NEEDLE, AND METHOD FOR PRODUCING HOLLOW MEDICAL NEEDLE**

(30) Priority: 29.10.2021 JP 2021178323
(71) Applicant: NIPRO CORPORATION, Kita-ku Osaka-shi, Osaka 531-8510 (JP)
(72) Inventor: MASUDA, Toshiaki, Osaka-shi, Osaka 531-8510 (JP); NAKAGAMI, Hiroyuki, Osaka-shi, Osaka 531-8510 (JP); SUZUKI, Ken, Osaka-shi, Osaka 531-8510 (JP)
(74) Representative: Slingsby Partners LLP
(86) International application number: PCT/JP2022/040626
(87) International publication number: WO 2023/074892

(57) **Abstract**

Provided is a medical hollow needle with a novel structure that allows the efficient passage of blood or other liquids. A medical hollow needle 10 has an inner lumen 12 penetrating it in a direction of a needle axis. An inner circumferential surface 16 of a needle tube 14 constituting a circumferential wall of the inner lumen 12 has a tapered surface 30 with a diameter becoming smaller toward a needle tip 18 formed by deep drawing. An arithmetic mean roughness Ra of the inner circumferential surface 16 in the direction of the needle axis is set as Ra≤1.0µm.

## Description

### TECHNICAL FIELD

The present invention relates to a medical hollow needle for use in, for example, artificial dialysis and a method for manufacturing the medical hollow needle.

### BACKGROUND ART

Conventionally, medical hollow needles used for injection and fluid transfusion, etc. have been manufactured by rounding a sheet of medical metal into a cylindrical shape and performing a multi-step extraction process on it. However, in this case, when the diameter of the hollow needle is small, the inner die cannot be inserted radially inward into the hollow needle, and the subsequent extraction process is performed without the inner die. Thus, the inner circumferential surface of the hollow needle may be wrinkled and the like, and the surface roughness of the inner circumferential surface may become rougher, which could hinder the smooth flow of the liquid. On the other hand, there is a technique for manufacturing the hollow needle by rounding a very thin sheet of medical metal into a cylindrical shape and joining the edges (e.g., Japanese Unexamined Patent Publication No. JP-A-2003-200218 (Patent Document 1)). In this case, since there is no need to perform the multi-step extraction process, it is possible to prevent wrinkles on the inner circumferential surface and the like.

### BACKGROUND ART DOCUMENTS

### PATENT DOCUMENTS

Patent Document 1: JP-A-2003-200218

### SUMMARY OF THE INVENTION

### PROBLEM THE INVENTION ATTEMPTS TO SOLVE

However, when the medical hollow needle is formed by rounding the sheet into the cylindrical shape, a joint portion is formed by the edges of the sheet in part of the medical hollow needle in the circumferential direction, and the surface roughness of the inner circumferential surface of the medical hollow needle becomes rough at the joint portion, which may cause resistance to the flow of liquid. Especially in the case of the medical hollow needle in which blood or other liquid flows inside, the joint portion was welded to ensure liquid tightness, thus it may be difficult to make the inner circumferential surface of the joint portion smooth.

Additionally, in the case of obtaining the hollow needle by the multi-step extraction process, as mentioned above, the surface roughness of the inner circumferential surface may become rough due to wrinkles, etc. on the inner circumferential surface, which may hinder the smooth flow of liquid.

The present invention has been developed in view of the above-described matters as the background, and it is an object of the present invention to provide a medical hollow needle with a novel structure which is able to efficiently flow a liquid such as blood. It is another object of the present invention to provide a method for manufacturing the medical hollow needle.

### MEANS FOR SOLVING THE PROBLEM

Hereinafter, preferred embodiments for grasping the present invention will be described. However, each preferred embodiment described below is exemplary and can be appropriately combined with each other. Besides, a plurality of elements described in each preferred embodiment can be recognized and adopted as independently as possible, or can also be appropriately combined with any element described in other preferred embodiments. By so doing, in the present invention, various other preferred embodiments can be realized without being limited to those described below.

A first preferred embodiment provides a medical hollow needle having an inner lumen penetrating it in a direction of a needle axis, comprising a needle tube, wherein an inner circumferential surface of the needle tube has a tapered surface with a diameter becoming smaller toward a needle tip formed by deep drawing, and an arithmetic mean roughness Ra of the inner circumferential surface in the direction of the needle axis is set as Ra≤1.0µm.

With the medical hollow needle structured according to the present preferred embodiment, since the needle tube constituting the circumferential wall of the inner lumen is formed by deep drawing, unevenness constituting surface roughness is reduced on the inner circumferential surface of the needle tube to decrease pressure loss, thereby ensuring a large flow rate of the fluid flowing through the inner lumen and reducing damages to blood cells, for example when blood flows through the inner lumen.

The tapered surface is set on the inner circumferential surface of the needle tube of the medical hollow needle. The tapered surface is formed by deep drawing, and changes in the inner diameter dimension can be suppressed to such an extent as the flow of liquid in the inner lumen is hardly affected. Since the tapered surface becomes smaller in diameter as it goes toward the distal end, the flow rate is stabilized, especially when the liquid flows through the inner lumen toward the distal end (the needle tip side).

The arithmetic mean roughness Ra of the inner circumferential surface of the needle tube in the direction of the needle axis is 1.0µm or less, and the inner circumferential surface of the needle tube, which is the inner surface of the circumferential wall of the inner lumen, is a smooth surface with small irregularities, so that the flow of liquid in the inner lumen is less likely to cause turbulent flow due to the surface roughness of the inner circumferential surface, effectively stabilizing the flow rate and reducing damages to blood cells.

A second preferred embodiment provides the medical hollow needle according to the first preferred embodiment, wherein an inner diameter of a distal end of the needle tube on a needle tip side is smaller than an inner diameter of a proximal end of the needle tube on an opposite side to the needle tip, and the distal end and the proximal end are connected by a stepped part whose diameter decreases toward the needle tip side, and the inner circumferential surface of the needle tube has a greater inclination angle relative to the needle axis at the stepped part than at the distal end and at the proximal end, and the tapered surface is constituted by the inner circumferential surface at the distal end and the proximal end.

With the medical hollow needle constructed according to the present preferred embodiment, the inner diameter of the distal end is smaller than that of the proximal end, which ensures the large flow rate in the inner lumen when the liquid flows through the inner lumen from the proximal end side to the distal end side (the needle tip side), for example.

Providing the stepped part connecting the distal end and the proximal end allows a large degree of freedom in setting the difference in inner diameter dimension between the distal end and the proximal end. In addition, the stepped part has a diameter decreasing as it goes toward the distal end, so that the flow is hardly dammed by the stepped part, thereby allowing a smooth flow.

A third preferred embodiment provides the medical hollow needle according to the first preferred embodiment, wherein the inner circumferential surface of the needle tube is the tapered surface with an approximately constant inclination angle relative to the needle axis over an entire length thereof in the direction of the needle axis.

With the medical hollow needle structured according to the present preferred embodiment, the inner circumferential surface of the needle tube is the tapered surface having the approximately constant inclination angle over the entire length in the direction of the needle axis, thereby stabilizing the flow rate and avoiding damages to blood cells more effectively.

A fourth preferred embodiment provides the medical hollow needle according to any one of the first through third preferred embodiments, wherein a molding trace extending in the direction of the needle axis is present on the inner circumferential surface of the needle tube in a micrograph.

In the medical hollow needle according to the present preferred embodiment, wherein the molding trace extending in the direction of the needle axis is present on the inner circumferential surface in a micrograph, the inner circumferential surface of the needle tube is molded, so that the surface roughness is suppressed, and the distal end of the convex part of the concave-convex surface roughness of the inner circumferential surface is made as crushed by a punch or other molding die. This makes it easy to suppress the arithmetic mean roughness Ra of the inner circumferential surface in the direction of the needle axis to 1.0µm or less so as to achieve the effect of the present invention as described above.

A fifth preferred embodiment provides the medical hollow needle according to any one of the first through fourth preferred embodiments, wherein the needle tube does not include any joint portion in a circumferential direction
With the medical hollow needle structured according to the present preferred embodiment, since the needle tube does not have any joint portion in the circumferential direction, it is possible to avoid the surface roughness of the inner circumferential surface from becoming locally large at the joint portion.

A sixth preferred embodiment provides the medical hollow needle according to any one of the first through fifth preferred embodiments, wherein an inclination angle of the tapered surface relative to the needle axis is greater than 0 degrees and not greater than 5 degrees.

With the medical hollow needle structured according to the present preferred embodiment, the inclination angle of the tapered surface relative to the needle axis is greater than 0 degrees and not greater than 5 degrees. This makes it possible to ensure the extraction taper of the deep drawing inner die (punch) and set a small rate of change of the inner diameter dimension in the direction of the needle axis, thereby suppressing the effect of the tapered surface on the liquid flow in the inner lumen.

A seventh preferred embodiment provides a method for manufacturing the medical hollow needle according to any one of the first through sixth preferred embodiments, comprising: a pressing process of performing a multi-step deep drawing on a blank metal plate to form a bottomed tube; a needle tip forming process of cutting the tube at a side of a deep-drawn bottom thereof to form the needle tip.

According to the method for manufacturing the medical hollow needle according to the present preferred embodiment, the bottomed tube is formed by the multi-step deep drawing on the blank metal plate, so that it is possible to obtain the needle tube without the joint portion in the circumferential direction. The inner circumferential surface of the tube is set as a tapered surface that expands as it goes toward the proximal end. This makes it possible to prevent the punch (inner die) for molding the inner circumferential surface of the tube from being stuck in the inner lumen of the tube. In addition, the punch is inserted radially inward into the tube for performing deep drawing. This reduces the unevenness that constitutes the surface roughness of the inner circumferential surface, and makes the sharp shape of the distal end of the convex part flatter, thereby reducing the surface roughness of the inner circumferential surface.

Also, the needle tip can be easily formed by cutting the bottom side of the bottomed tube obtained by deep drawing. By cutting off the bottom of the tube when forming the needle tip, it is possible to form the inner lumen of the needle tube that penetrates it in the direction of the needle axis.

### EFFECT OF THE INVENTION

According to the present invention, it is possible to provide the medical hollow needle capable of efficiently flowing liquids such as blood.

### BRIEF DESCRIPTION OF THE DRAWINGS

FIG. 1 is a plan view of a medical hollow needle as a first practical embodiment of the present invention.
FIG. 2 is a cross sectional view taken along line 2-2 of FIG. 1.
FIG. 3 is an enlarged transverse cross sectional view of the hollow medical needle of FIG. 1, taken along line 3-3 of FIG. 2.
FIGS. 4A to 4H are views suitable for explaining the manufacturing processes of the medical hollow needle shown in FIG. 1.
FIG. 5 is a micrograph of an inner circumferential surface of the medical hollow needle shown in FIG. 1.
FIG. 6 is a micrograph of an inner circumferential surface of a conventional medical hollow needle.
FIG. 7 is a graph of the center mean roughness on the inner circumferential surface of the medical hollow needle shown in FIG. 5.
FIG. 8 is a graph of the center mean roughness on the inner circumferential surface of the medical hollow needle shown in FIG. 6.
FIG. 9 is a longitudinal cross sectional view of a medical hollow needle as a second practical embodiment of the present invention.

### EMBODIMENTS FOR CARRYING OUT THE INVENTION

Practical embodiments of the present invention will be described below in reference to the drawings.

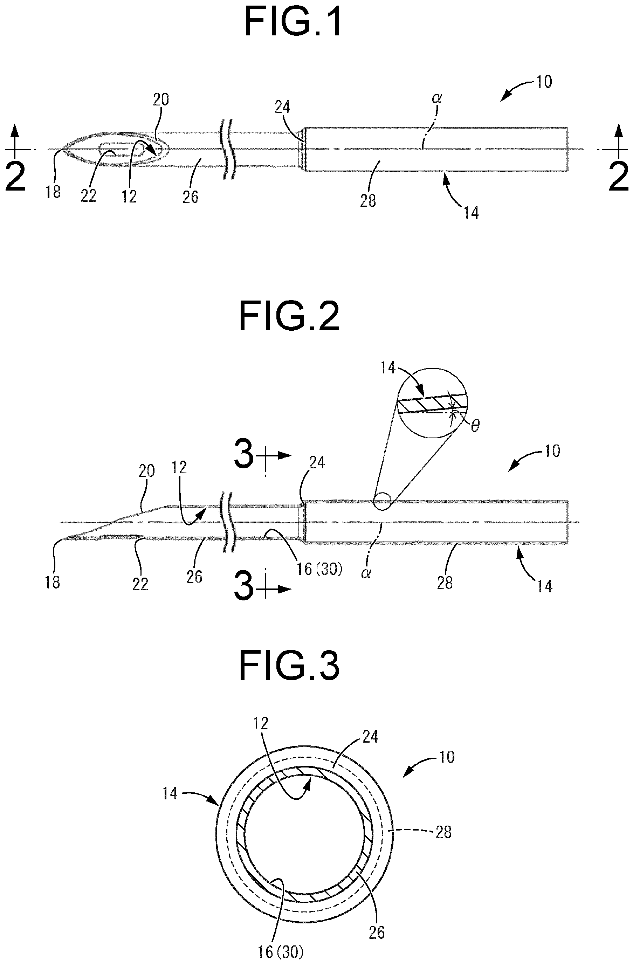

FIGS. 1 to 3 show a medical hollow needle 10 as a first practical embodiment of the present invention. The medical hollow needle 10 has an inner lumen 12 penetrating it in the axial direction and is constituted by a tubular needle tube 14 constituting the peripheral wall of the inner lumen 12. In the following explanation, in principle, the distal end side means the left side in FIG. 1, which is the side of a needle tip 18 described below, while the proximal end side means the right side in FIG. 1, which is the side opposite to the needle tip 18. In addition, as a general rule, the up-down direction means the up-down direction in FIG. 2.

The needle tube 14 has a small-diameter cylindrical shape as a whole and, as shown in FIG. 3, has no joint portion in the circumferential direction. That is, the needle tube 14 is not made of a plate material rounded into a tubular shape, and there is no joint portion formed by the both edges in the circumferential direction when the plate material is rounded into the tubular shape. Therefore, the needle tube 14 has an approximately uniform inner circumferential surface 16 over the entire circumference. The needle tube 14 without any circumferential joint portion can be obtained, for example, by a multi-step pressing process (deep drawing), as described below.

As shown in FIGS. 1 and 2, the needle tube 14 has the sharp needle tip 18 at one end in the direction of the needle axis (the left-right direction in FIG. 1, the length direction). The needle tip 18 is provided, for example, by cutting the end of the needle tube 14 into a shape inclined to a needle axis α to form a blade surface 20. The blade surface 20 of this practical embodiment has different inclination angles to the needle axis α on the distal end side and the proximal end side, with the inclination angle being greater on the distal end side. For example, the inclination angle of the blade surface 20 to the needle axis α is not smaller than 15 degrees and not greater than 30 degrees on the distal end side, while the inclination angle is not smaller than 10 degrees and not greater than 20 degrees on the proximal end side. The blade surface 20 is not limited to a specific shape. For example, the blade surface 20 may be constituted by a single plane, three or more planes, or one or multiple curved surfaces.

In the distal end of the needle tube 14, there is formed a through hole 22 that passes through in a direction perpendicular to the needle axis α. This efficiently ensures the flow rate of liquid such as blood flowing into or out of the inner lumen 12, for example, when the medical hollow needle 10 punctures a blood vessel. In this practical embodiment, the through hole 22 is an oval shape which is elongated in the direction of the needle axis to ensure a space-efficient opening area, but the shape is not limited. The through hole 22 is not essential and can be omitted.

The needle tube 14 is formed so that the wall thickness decreases toward the distal end. The needle tube 14 has a stepped part 24 in the middle of its length direction, and a distal end 26 located on the needle tip 18 side of the stepped part 24 has a smaller diameter than a proximal end 28 located on the other side of the stepped part 24 opposite to the needle tip 18. The stepped part 24 serves as a fixation portion to the needle hub or the like, which is not shown, and it is fixed to the needle hub or the like by means of an adhesive, for example. The stepped part 24 has a larger outer diameter than that of the distal end 26, thus providing a larger adhesive area. It is desirable that the distal end 26 and the proximal end 28 should have a difference of 0.6 mm or less in inner diameter dimension. For example, the distal end 26 has an inner diameter dimension corresponding to 16 G (1.45 mm), while the proximal end 28 has an inner diameter dimension corresponding to 14 G (1.90 mm). The larger inner diameter of the proximal end 28 ensures the larger flow rate in the inner lumen 12. It is desirable that the distal end 26 and the proximal end 28 should have a difference of 0.6 mm or less in outer diameter dimension. For example, the distal end 26 has an outer diameter dimension corresponding to 16 G (1.65 mm), while the proximal end 28 has an outer diameter dimension corresponding to 14 G (2.10 mm). The smaller outer diameter of the distal end 26 reduces pain during puncture. The length dimension of the distal end 26 in the direction of the needle axis is set as appropriate according to the puncture depth for each needle application, and should have at least such a length that the stepped part 24 does not touch the patient's skin during the puncture. In this practical embodiment, the length dimension of the distal end 26 in the direction of the needle axis is larger than the length dimension of the proximal end 28 in the direction of the needle axis, and the difference is suitably 1.5 times or more. Considering that the blood vessel is punctured with the distal end 26, the length dimension of the distal end 26 in the direction of the needle axis should be 25 mm or more. Considering that the proximal end 28 or the stepped part 24 serves as the fixation portion to the needle hub, which is not shown, the length dimension in the direction of the needle axis from the distal end of the distal end 26 to the base end of the proximal end 28 (the total length of the medical hollow needle 10) should be 30 mm or more, desirably. In this practical embodiment, the stepped part 24, which is a part of the medical hollow needle 10, is used as the fixation portion to the needle hub. However, for example, another member can be joined to the proximal end side of the medical hollow needle of the present invention to serve as the fixation portion to the needle hub. In this case, the total length of the medical hollow needle may be less than 30 mm because the medical hollow needle does not include the fixation portion to the needle hub. The above mentioned separate member is not limited in its formation method. For example, it may be formed by deep drawing, or it may be formed by rounding a plate material into a tubular shape and then thinning it by an extraction process.

The length dimension of the stepped part 24 in the direction of the needle axis is shortened relative to the length dimensions of the distal end 26 and the proximal end 28 in the same direction. Additionally, the stepped part 24 can be eliminated by the needle tube 14 having a tapered shape that is tapered approximately constant over the entire length of the needle tube 14 from the distal end to the proximal end.

The stepped part 24 is tapered with a diameter gradually decreasing toward the needle tip 18, and the distal end 26 with a smaller diameter and the proximal end 28 with a larger diameter are connected via the stepped part 24. The distal end 26 and the proximal end 28 are each tapered with a diameter gradually becoming smaller toward the needle tip 18. The inner circumferential surface 16 of the needle tube 14 has a tapered surface 30 constituted by the inner circumferential surfaces of the distal end 26 and the proximal end 28. The tapered surface 30 is formed by deep drawing, and, for example, when the needle tube 14 is formed by deep drawing, it is set as the extraction taper of the punch, which is the inner die. It is desirable that an inclination angle θ of the tapered surface 30 relative to the needle axis α is greater than 0 degrees and is not greater than 5 degrees, suitably not greater than 1 degree, and more suitably not greater than 0.7 degrees. The smaller the taper angle θ is, the less the patient's skin is pushed open during the puncture, and the less pain accompanies the puncture. As the inclination angle θ of the tapered surface 30, it is possible to set an angle outside the above-mentioned angle range. For example, an angle greater than 5 degrees may be set. The inclination angle of the inner circumferential surface of the stepped part 24 relative to the needle axis α is greater than the inclination angle θ of the tapered surface 30 in this practical embodiment, e.g., not smaller than 15 degrees and not greater than 60 degrees. However, it is also possible to set an angle outside the angle range, for example, 15 degrees or less, considering ease of manufacture, degree of turbulence, etc. FIG. 2 shows a partially enlarged view of a part of the needle tube 14, in which a hypothetical line parallel to the needle axis α is shown as a dotted line to indicate the inclination angle θ of the tapered surface 30 constituting the inner circumferential surface 16 of the needle tube 14 relative to the needle axis α. In this enlarged view, the inclination angle θ of the tapered surface 30 is shown exaggeratedly large for ease of viewing.

The inner circumferential surface 16 of the needle tube 14 has an arithmetic mean roughness Ra in the direction of the needle axis of 1.0µm or less (Ra≤1.0µm). More preferably, the arithmetic mean roughness Ra of the inner circumferential surface 16 in the direction of the needle axis is 0.7µm or less (Ra ≤ 0.7µm), and the closer to 0 the arithmetic mean roughness Ra is, the more desirable it is. In short, the inner circumferential surface 16 of the needle tube 14 is a smooth surface with small irregularities. The needle tube 14 should have an arithmetic mean roughness Ra of the outer circumferential surface in the direction of the needle axis of 1.0µm or less (Ra ≤ 1.0µm), more preferably 0.7µm or less (Ra ≤ 0.7µm). As is generally known, the arithmetic mean roughness Ra is calculated as the mean value of the distance from the reference line of the unevenness, using the mean value of the unevenness that constitutes the surface roughness as the reference line.

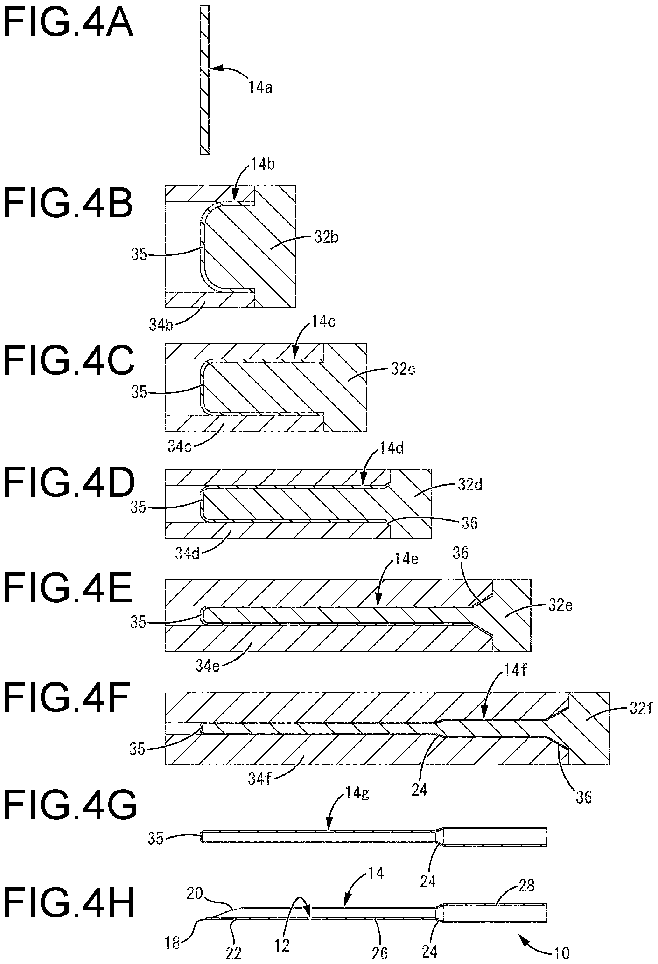

The medical hollow needle 10 having such a structure can be obtained by the manufacturing method including each of the processes shown in FIGS. 4A to 4H. FIGS. 4A to 4H show each process constituting the manufacturing method of the medical hollow needle 10 in order from the top in the figure. The manufacturing method of the medical hollow needle 10 includes (a) the blank plate preparation process, (b) the first pressing process, (c) the second pressing process, (d) the third pressing process, (e) the fourth pressing process, (f) the fifth pressing process, (g) flange cutting process, and (h) needle tip forming process. Since FIGS. 4A to 4H are views for explaining each process of the manufacturing method, the shapes and dimensions of a blank metal plate 14a, first through sixth molded articles 14b to 14g, and the needle tube 14, for example, are not strictly accurate.

First, in the blank plate preparation process shown in FIG. 4A, the blank metal plate 14a is prepared. The blank metal plate 14a is made of medical metal material such as medical stainless steel, and has a shape of substantially flat plate.

Next, a pressing process of pressing the prepared blank metal plate 14a using a punch 32 as the inner die and a die 34 as the outer die is performed. The pressing process is performed in stages over a number of times, and in this practical embodiment, five pressing processes are performed from the first to the fifth. The punches used in each the first through fifth pressing processes are marked with codes 32b to 32f, and the dies used in each the first through fifth pressing processes are marked with codes 34b through 34f. From the first pressing process performed first to the fifth pressing process performed last, the outer diameters of punches 32b to 32f, which define the inner diameters of the first to fifth molded articles 14b to 14f, and the inner diameters of dies 34b to 34f, which define the outer diameters of the first to fifth molded articles 14b to 14f, are gradually reduced.

Specifically, in the primary pressing process (the first pressing process in FIG. 4B), the plane blank metal plate 14a is subject to deep drawing by the punch 32b and the die 34b to obtain the bottomed tubular first molded article 14b with a large diameter and a small depth dimension. The bottom 35 of the first molded article 14b has an approximately disk shape, and the outer circumference has a curved cross-sectional shape that is smoothly continuous with the circumferential wall. Furthermore, in the second pressing process in FIG. 4C, the second molded article 14c with a smaller diameter and a larger depth dimension than those of the first molded article 14b is obtained using the punch 32c and the die 34c. In the third pressing process in FIG. 4D, the third molded article 14d with a smaller diameter and a larger depth dimension than those of the second molded article 14c is obtained using the punch 32d and the die 34d. Furthermore, in the fourth pressing process in FIG. 4E, the fourth molded article 14e with a smaller diameter and a larger depth dimension than those of the third molded article 14d is obtained using the punch 32e and the die 34e. In the final pressing process (the fifth pressing process in FIG. 4F), the fifth molded article 14f with a smaller diameter and a larger depth dimension than those of the fourth molded article 14e is obtained using the punch 32f and the die 34f. Thus, the first through fifth pressing processes gradually decrease the inner and outer diameters of the needle tube 14 and gradually increase the length in the direction of the needle axis to obtain the fifth molded article 14f as a tube that can be used as the medical hollow needle 10.

The fifth molded article 14f obtained at the completion of the fifth pressing process has a generally bottomed tubular shape with the distal end closed by the deep-drawn bottom 35, and a flanged part 36 that expands as it goes toward the proximal end is integrally provided at the base end. In this practical embodiment, the stepped part 24 is formed in the fifth pressing process, which is the forming process of the fifth molded article 14f.

The first molded article 14b to the fifth molded article 14f obtained in the respective pressing processes are tapered with the circumferential wall expanding toward the proximal end opening, and especially the inner circumferential surface is an inclined surface expanding toward the proximal end opening over the entire area including the tapered surface 30. Therefore, the punches 32b to 32f can be easily pulled out from the first to fifth molded articles 14b to 14f toward the proximal end after molding.

Next, in the flange cutting process shown in FIG. 4G, the flanged part 36 of the fifth molded article 14f is cut to obtain the sixth molded article 14g as a tube. The sixth molded article 14g with the flanged part 36 cut out has a smaller maximum outer diameter dimension than that of the fifth molded article 14f with the flanged part 36, so that the space required for transportation and storage can be reduced.

Next, in the needle tip forming process of FIG. 4H, the sixth molded article 14g, for which the flanged part 36 is cut out, is made into the needle tube 14 (the medical hollow needle 10) having the blade surface 20 and the needle tip 18 at the distal end, by cutting the distal end having the bottom 35 at an angle. The medical hollow needle 10 according to the present practical embodiment can be manufactured by the manufacturing process described above. In this practical embodiment, the through hole 22 is formed in the distal end of the needle tube 14 after the needle tip forming process is completed. The formation of the needle tip 18 by cutting the distal end and the cutting of the flanged part 36 can be carried out as a single process.

Thus, by forming the needle tube 14 in a tubular shape from a plate material (the blank metal plate 14a) by a multi-step deep drawing process, the needle tube 14 with a substantially uniform circumferential wall without a joint portion in the circumferential direction can be obtained. Hence, the surface roughness of the inner circumferential surface 16 of the needle tube 14, which is the circumferential wall surface of the inner lumen 12, does not locally increase at the joint portion.

The inner circumferential surface 16 of the needle tube 14 stretched by the first to fifth pressing processes has an arithmetic mean roughness Ra in the direction of the needle axis of 1.0µm or less (Ra≤1.0µm), more suitably 0.7µm or less (Ra≤0.7µm), which is smaller than that of a conventional needle tube made by rounding a plate material and reducing its diameter by extraction molding. This suppresses the flow resistance of liquid such as blood flowing in the inner lumen 12, and it is possible to obtain a stable and efficient flow rate and to reduce damages to blood cells.

Moreover, the distal end of the convex part, which constitutes the concave/convex surface roughness on the inner circumferential surface 16 of the needle tube 14, is flattened by contact against the punch 32 during multiple pressings. The distal end of the convex part has a surface shape, thus reducing the sharpness of the convex part's distal end. As a result, when liquid flows through the inner lumen 12 of the needle tube 14, the resistance due to the minute irregularities of the inner circumferential surface 16 is reduced, and efficient flow of the liquid, etc. is achieved.

The needle tube 14 formed by deep drawing has the tapered surface 30 with the inner circumferential surface 16 set at an inclination in the direction of widening toward the proximal end to enable the punches 32b to 32f to be easily extracted from the inner circumference of the first to fifth molded articles 14b to 14f in the pressing processes. The inclination angle (taper angle) θ of this tapered surface 30 relative to the needle axis α is set to be as small as 5 degrees or less (preferably 1 degree or less, more preferably 0.7 degrees or less), so that the effect on the flow of liquid through the inner lumen 12 is substantially avoided.

In this practical embodiment, the stepped part 24 is provided in the middle of the needle tube 14 in the length direction, and the inner diameter of the distal end 26, which is located on the distal end side of the needle tube 14 relative to the stepped part 24, is smaller than that of the proximal end 28, which is located on the proximal end side thereof relative to the stepped part 24. This ensures, for example, a large flow rate when liquid flows from the proximal end side to the distal end side of the medical hollow needle 10. Furthermore, in the medical hollow needle 10, the distal end 26, which is the part that punctures the patient, is smaller in diameter than the proximal end 28, thereby reducing pain during puncture.

The needle gauge G, which indicates the thickness of the medical hollow needle 10, is set, for example, by the mean value of the thickness (outer and inner diameter dimensions) of the distal end 26, which is the part that punctures the patient. The proximal end 28 may be exposed externally, or it may be inserted into, for example, a needle hub or a syringe cylinder or the like, which is not shown, to serve as a fixation portion to the needle hub or the like. Preferably, the distal end 26 is the portion exposed from the needle hub, the syringe, and the adhesive for adhering to them, etc., and the axial length of the distal end 26 is the effective length of the medical hollow needle 10.

Since the distal end 26 and the proximal end 28, which have different inner diameter dimensions, are connected via the stepped part 24, the inner diameter dimensions of the distal end 26 and the proximal end 28 can be set with a large degree of freedom. Thus, for example, it is also possible to make the distal end 26, which pierces the patient, small enough in diameter to minimize pain at the time of puncture, while making the proximal end 28 large enough in diameter to ensure the flow rate of liquid in the inner lumen 12. Furthermore, the stepped part 24 has a tapered shape with a diameter decreasing toward the distal end, preventing a sudden change in the cross section of the stepped part 24, thereby reducing adverse effects on the flow of the liquid through the inner lumen 12.

The medical hollow needle 10 is preferably used, for example, for the retransfusion port and blood removal port of artificial dialysis circuits and the administration port, which is the patient-side distal end of fluid transfusion circuits, and the like. In particular, the medical hollow needle 10 is preferably used for puncture portions in artificial dialysis circuits and blood transfusion circuits through which blood flows inside. Specifically, for example, the medical hollow needle 10 can be suitably used as a needle for artificial dialysis. This can realize minimally invasive dialysis and fluid transfusion, since owing to the smooth flow of liquid, damages to blood cells and entrapment of air bubbles, etc. during artificial dialysis and fluid transfusion (including blood transfusion) hardly occur.

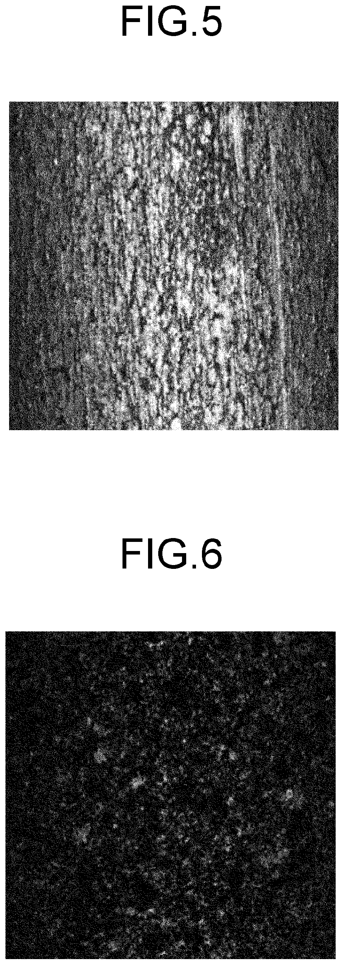

The fact that the inner circumferential surface 16 of the medical hollow needle 10 of the present invention is a smooth surface with a smaller surface roughness than that of the inner circumferential surface of a conventional medical hollow needle is confirmed by the micrographs shown in FIGS. 5 and 6. FIG. 5 is a micrograph of the inner circumferential surface 16 of the medical hollow needle 10 according to the present invention, while FIG. 6 is a micrograph of the inner circumferential surface of the conventional medical hollow needle. In FIGS. 5 and 6, the up-down direction in the drawings is the direction of the needle axis, and the left-right direction in the drawings is the circumferential direction. The conventional medical hollow needle relating to FIG. 6 is formed by a conventional manufacturing method, in which a flat plate material is rounded into a tubular shape, both edges of which are butted in the circumferential direction and jointed by welding, and then it is thinned to a prescribed thickness by a multi-step extraction process. The micrographs in FIGS. 5 and 6 are taken using an "OLS4100" laser microscope manufactured by Olympus Corporation with a 20x objective lens and an observation magnification of 432x.

In the micrograph in FIG. 5, molding traces extending in the direction of the needle axis are present on the inner circumferential surface 16 of the medical hollow needle 10. Such molding traces on the inner circumferential surface 16 are formed when the punches 32b-32f are inserted or removed radially inwardly in the direction of the needle axis in relation to the first to fifth molded articles 14b to 14f during the pressing process, because the convex parts constituting the surface roughness of each inner circumferential surface of the first to fifth molded articles 14b to 14f are crushed or shaved off by the contact of the punches 32b-32f. Therefore, the molding traces on the inner circumferential surface 16 of the needle tube 14 can be checked in the micrograph by the fact that the convex part constituting the surface roughness of the inner circumferential surface 16 of the needle tube 14 has a longitudinal shape extending in the direction of the needle axis, and that the distal end of the convex part has a flatter surface shape with a smaller degree of sharpness. On the other hand, in the micrograph shown in FIG. 6, which shows a needle tube formed by extraction molding without an inner die (plug), no clear molding traces can be seen, and the distal end of the convex part that constitutes the surface roughness has a sharp, spot-like (point-like) shape that does not extend in the direction of the needle axis.

Comparing FIGS. 5 and 6, due to this difference in the molding traces, the distal end of the convex part protruding radially inward, which constitutes the surface roughness, has a surface shape in FIG. 5, and, in contrast, the distal end of the convex part has a sharply pointed shape in FIG. 6. This difference in shape of the inner circumferential surface suggests that the medical hollow needle 10 of the present invention can achieve a smoother flow of liquid than the conventional medical hollow needle.

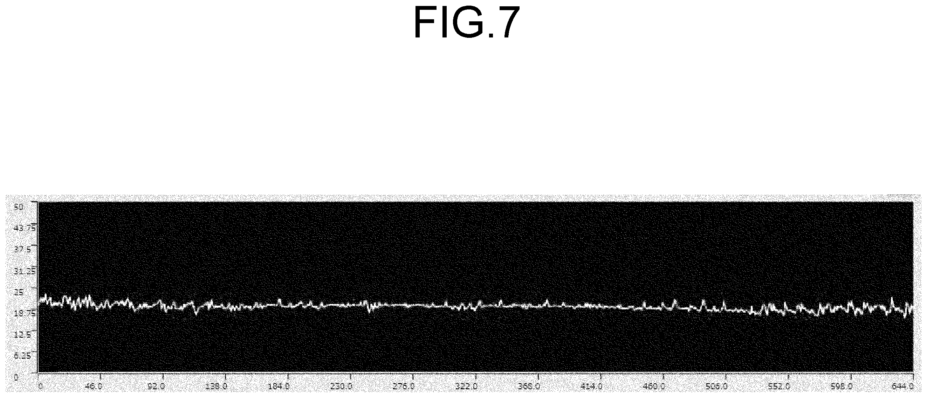

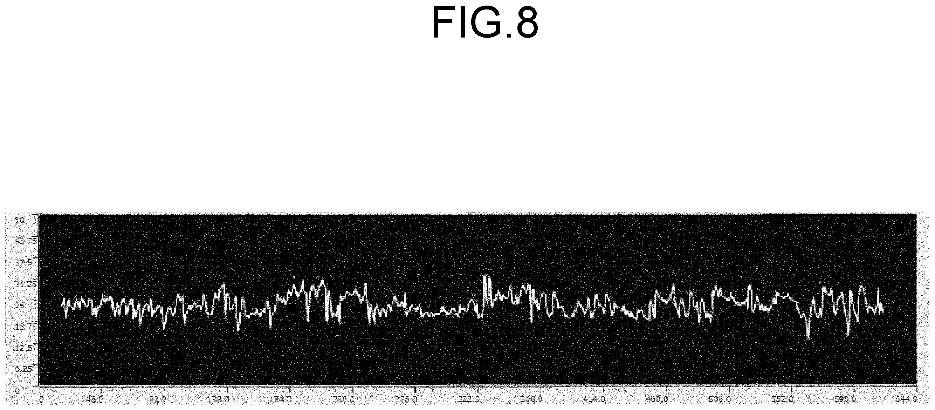

The surface roughness of the inner circumferential surface was also measured by laser when the micrographs in FIGS. 5 and 6 were taken, and the measurement results of the surface roughness (size of unevenness) of the inner circumferential surface are shown in FIGS. 7 and 8. FIG. 7 shows the measurement results of the unevenness of the inner circumferential surface 16 of the medical hollow needle 10 of the present invention, while FIG. 8 shows the measurement results of the unevenness of the inner circumferential surface of the conventional medical hollow needle. The graphs in FIGS. 7 and 8 show the line roughness in the direction of the needle axis. In the graphs in FIGS. 7 and 8, the transverse axis indicates a position in the direction of the needle axis of the medical hollow needle, while the vertical axis indicates the height dimension from the reference position to the inner circumferential surface of the medical hollow needle. Therefore, in the graphs of FIGS. 7 and 8, there is shown in the transverse axis direction that the smaller the range of variation in the vertical axis direction is, the smaller and smoother the unevenness of the inner circumferential surface of the medical hollow needle is.

Comparing FIGS. 7 and 8, the graph of the measurement results shown in FIG. 7 has a smaller range of variation in the vertical axis direction than the graph of the measurement results shown in FIG. 8. Thus, it is confirmed also by actual measurement that the inner circumferential surface 16 of the medical hollow needle 10 of the present invention is smoother with less unevenness than the inner circumferential surface of the conventional medical hollow needle. For the needle tube shown in FIG. 5, it is confirmed that the surface roughness was approximately as good in the circumferential direction as in the direction of the needle axis.

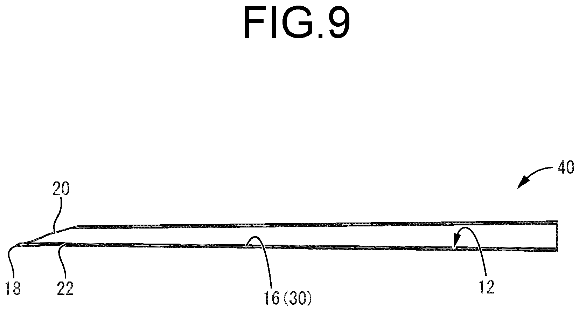

FIG. 9 shows a medical hollow needle 40 as a second practical embodiment of the present invention. In the following description, explanations about components and parts that are substantially the same as those of the first practical embodiment are omitted, by providing them with the same symbols in the drawing.

Compared with the medical hollow needle 10 of the first practical embodiment, the medical hollow needle 40 is not provided with the stepped part 24, and the entire inner circumferential surface 16 is the tapered surface 30. In addition, the inclination angle θ of the inner circumferential surface 16 relative to the needle axis α is approximately constant over the entire length in the direction of the needle axis. According to the medical hollow needle 40 of this practical embodiment, there is no sudden change in the inner diameter dimension and the flow of liquid in the inner lumen 12 is more stable, which can further stabilize the flow rate and reduce damage to blood cells in the blood flowing through the inner lumen 12.

Although the practical embodiments of the present invention have been described in detail above, this invention is not limited by that specific description. For example, the first practical embodiment shows an example in which the inclination angle θ of the tapered surface 30 constituting the inner circumferential surface 16 of the medical hollow needle 10 is almost constant except at the portion where the stepped part 24 is formed. However, the inclination angle θ of the tapered surface 30 may vary in a stepwise manner or continuously. Specifically, for example, in the medical hollow needle 10 of the first practical embodiment, the inclination angle of the inner circumferential surface of the distal end 26 and that of the proximal end 28 can be different from each other. The inclination angle θ of the inner circumferential surface 16 may vary in the circumferential direction. When the inclination angle θ of the tapered surface 30 set on the inner circumferential surface 16 of the medical hollow needle 10 varies in the direction of the needle axis or in the circumferential direction, it is desirable that the minimum inclination angle in the tapered surface 30 is greater than 0 degrees and the maximum inclination angle is 5 degrees or less (suitably 1 degree or less, more suitably 0.7 degrees or less).

In the medical hollow needle 10, the stepped parts 24 may be provided at two or more locations in the direction of the needle axis. In this case, the inner circumferential surface of the middle portion of the medical hollow needle 10 located between the adjacent stepped parts 24, 24 in the direction of the needle axis should be the tapered surface 30, as well as the distal end 26 and the proximal end 28, and it should have a tapered shape having a larger diameter at the proximal end side, with the same inclination angle as the distal end 26 and the proximal end 28.

The above-mentioned practical embodiment shows, as an example, the method for manufacturing the medical hollow needle 10 comprising the first through fifth pressing processes. However, the number of pressing processes is not limited to five, but may be four or less, or six or more. In particular, the number of pressing processes may be increased when a thinner medical hollow needle 10 is manufactured.

In the aforesaid practical embodiment, by cutting the bottom 35 side of the sixth molded article 14g, which has a bottomed tubular shape, formation of the needle tip 18 and the blade surface 20 and penetration of the inner lumen 12 are performed at the same time. Alternatively, for example, a hole may be formed through the bottom 35 of the sixth molded article 14g with a punch to complete penetration of the inner lumen 12 and then cutting may be performed to form the needle tip 18 and the blade surface 20.

### KEYS TO SYMBOLS

10: Medical hollow needle (first practical embodiment); 12: Inner lumen; 14: Needle tube; 14a: Blank metal plate; 14b: First molded article; 14c: Second molded article; 14d: Third molded article; 14e: Fourth molded article; 14f: Fifth molded article (tube); 14g: Sixth molded article (tube); 16: Inner circumferential surface; 18: Needle tip; 20: Blade surface; 22: Through hole; 24: Stepped part; 26: Distal end; 28: Proximal end; 30: Tapered surface; 32: Punch; 34: Die; 35: Bottom; 36: Flanged part; 40: Medical hollow needle (second practical embodiment); α: Needle axis; θ: Inclination angle

## Claims

1. A medical hollow needle having an inner lumen penetrating it in a direction of a needle axis, comprising a needle tube, wherein
an inner circumferential surface of the needle tube has a tapered surface with a diameter becoming smaller toward a needle tip formed by deep drawing, and
an arithmetic mean roughness Ra of the inner circumferential surface in the direction of the needle axis is set as Ra≤1.0µm.

2. The medical hollow needle according to claim 1, wherein
an inner diameter of a distal end of the needle tube on a needle tip side is smaller than an inner diameter of a proximal end of the needle tube on an opposite side to the needle tip, and the distal end and the proximal end are connected by a stepped part whose diameter decreases toward the needle tip side, and
the inner circumferential surface of the needle tube has a greater inclination angle relative to the needle axis at the stepped part than at the distal end and at the proximal end, and the tapered surface is constituted by the inner circumferential surface at the distal end and the proximal end.

3. The medical hollow needle according to claim 1, wherein
the inner circumferential surface of the needle tube is the tapered surface with an approximately constant inclination angle relative to the needle axis over an entire length thereof in the direction of the needle axis.

4. The medical hollow needle according to any one of claims 1-3, wherein a molding trace extending in the direction of the needle axis is present on the inner circumferential surface of the needle tube in a micrograph.

5. The medical hollow needle according to any one of claims 1-4, wherein an inclination angle of the tapered surface relative to the needle axis is greater than 0 degrees and not greater than 5 degrees.

6. A method for manufacturing the medical hollow needle according to any one of claims 1-5, comprising:
a pressing process of performing a multi-step deep drawing on a blank metal plate to form a bottomed tube;
a needle tip forming process of cutting the tube at a side of a deep-drawn bottom thereof to form the needle tip.
